# EUROPEAN PATENT SPECIFICATION

(11) **EP 1 676 591 B1**
(45) Date of publication and mention of the grant of the patent: **01.08.2018**
(21) Application number: 05257209.6
(22) Date of filing: 23.11.2005
(51) Int. Cl.: A61L 27/54, A61L 27/34, A61L 27/38, A61L 27/56

(54) **Compositions and methods to create a vascularized environment for cellular transplantation**
Zusammensetzungen und Verfahren zur Herstellung einer vaskularisierten Umgebung für die Zelltransplantation
Compositions et méthodes pour la création d'un environnement vascularisé pour la transplantation cellulaire

(30) Priority: 24.11.2004 US 630633 P
(43) Date of publication of application: 05.07.2006
(73) Proprietor: LifeScan, Inc., Wayne, PA 19087 (US)
(72) Inventor: Rezania, Alireza, NJ 08844 (US); Xu, Jean, NJ 08844 (US); Ghabrial, Ragae, NJ 08828 (US)
(74) Representative: Kirsch, Susan Edith

(56) References cited:
- EP-A- 1 310 266
- EP-A- 1 466 633
- WO-A-01/30368
- WO-A-02/098475
- WO-A-03/080566
- US-A1- 2003 092 716
- US-A1- 2004 214 777
- WARNECKE, C ET AL.: "Activation of the hypoxia-inducible factor pathway and stimulation of angiogenesis by application of prolyl hydroxylase inhibitors" FASEB JOURNAL, vol. 17, no. 9, 2003, pages 1186-1188, XP002391446 US

## Description

### FIELD OF THE INVENTION

The present invention generally relates to biocompatible devices suitable for supporting and implanting cells into a mammal wherein the device has incorporated into it at least one pharmaceutical agent. In particular, the pharmaceutical agent is capable of increasing the amount of hypoxia-inducible factor 1 alpha protein in cells at the implant site.

### BACKGROUND OF THE INVENTION

There is a need to develop devices for transplanting cells, organoids, organs or tissue to create artificial organs when the patient's own organ function is lost or impaired due to disease or injury. Such devices are able to deliver the transplanted cells to a specific site within the recipient. The therapeutic applications for the transplanted cells can include, for example, hepatocytes for the treatment of liver failure, chromaffin cells for chronic pain, cells that produce clotting factors for hemophilia, islets or insulin producing cells for the treatment of diabetes, cells that produce nerve growth factors for neurodegenerative disease such as Parkinson's or Alzheimer's disease, and cardiovascular cells for the treatment of cardiovascular diseases.

Many have tried to overcome the limitations of cell transplantation by creating a three-dimensional matrix or support, to which cells can attach *in vitro.* However, the physical properties of the support limit diffusion of oxygen and nutrients to the cells, due to lack of sufficient vasculature to carry such nutrients and to remove wastes, resulting in an ischemic environment for the transplanted cells. Consequently, the cells quickly die or cease to function once they are implanted into the recipient.

Previous attempts have focused on incorporation of growth factors into the support. These factors promote an angiogenic response to enhance implant survival. This approach has shown marginal effectiveness only when combinations of growth factors, such as platelet derived growth factor (PDGF-BB) plus vascular endothelial growth factor (VEGF) or basic fibroblast growth factor (bFGF), are delivered from a three-dimensional construct.

EP 1 466 633 discloses implantable, biocompatible scaffolds containing a biocompatible, porous polymeric matrix, a biocompatible, porous, fibrous mat encapsulated by and disposed within said polymeric matrix, and a plurality of mammalian cells seeded within said tissue scaffold.

The main challenge in tissue engineering is that recapitulating the normal angiogenesis response required for new vessel formation is very difficult to achieve because the precise nature, dose, and sequence of growth factors needed for stable vessel formation is poorly defined.

Hypoxia is a critical factor in induction of angiogenesis. An early response to tissue hypoxia is induction of hypoxia inducible factor-1 (HIF-1), a basic helix-loop-helix (bHLH) PAS (Per/Arnt/Sim) transcriptional activator that mediates changes in gene expression in response to changes in cellular oxygen concentration (Wang et al (1995), PNAS 92, 5510-5514). HIF-1 is a heterodimer containing an oxygen-regulated alpha subunit (HIF-1α - GenBank accession number AAP88778) and a constitutively expressed beta subunit (HIF-1β - GenBank accession number AAH60838), also known as aryl hydrocarbon receptor nuclear transporter (ARNT). Levels of HIF-1α protein are elevated in most cells in response to hypoxia and HIF-1α is induced *in vivo* when animals are subjected to anemia or hypoxia. HIF-1α levels rise within a few hours after the onset of hypoxia and return to baseline under continued hypoxic conditions.

HIF-1 has been implicated in numerous cellular and developmental processes, including cell proliferation, angiogenesis, and cell cycle arrest. In oxygenated (normoxic) cells, HIF-1α subunits are rapidly degraded by a mechanism that involves ubiquitination by the von Hippel-Lindau tumor suppressor (pVHL) E3 ligase complex. Under hypoxic conditions, HIF-1α is not degraded, and an active HIF-1α/β complex accumulates in the nucleus and activates the expression of several genes including glycolytic enzymes, glucose transporter (GLUT)-1, erythropoietin (EPO), and vascular endothelial growth factor (VEGF). HIF-1α has also been associated with myocardial acute ischemia and early infarction, pulmonary hypertension, and inflammation. Although HIF-1α has been associated with tumor growth and metastasis, there is little indication that HIF-1 is directly involved in tumorigenesis. Hypoxic preconditioning, in which a target organ is subjected to brief periods of hypoxia, has been shown to protect both the myocardium and brain against hypoxic-ischemic injury. HIF-1α stabilization is closely associated with ischemia and is induced by preconditioning. Results of work described herein indicate that increasing HIF-1α levels in cells at the transplant site is effective in enhancing vascularization of the transplanted site and enhancing the survival of the transplanted cells.

The construct of the present invention preferably provides a biodegradable support loaded with desirable agent(s), designed to increase the vasculature surrounding the construct leading to enhanced function of the construct.

### SUMMARY OF THE INVENTION

The current invention avoids the problems encountered by researchers in the past by using a device to create a vascularized transplant site within a mammal that improves the survivability of therapeutic cells. The current invention provides an implant according to claim 1, for the transplantation of cells, tissues, organoids, or organs for the effective treatment of a disease or injury. The implant comprises a porous support, that contains a pharmaceutical agent or agents that are designed to create a vascularized bed around and within the implant. The vascularized bed can significantly enhance the survival of transplanted cells, tissues, organoids or organs that are contained within the implant. The pharmaceutical agents include factors that can stabilize the alpha subunit of HIF (Hypoxia Inducible factor α). The term "stabilizing the alpha subunit of HIF" includes increasing expression of HIF-1α protein as well as preventing degradation of RNA encoding HIF-1α or degradation of HIF-1α itself. One class of agents that can stabilize HIF-1α is inhibitors of prolyl hydroxylase, the enzyme involved in the degradation of HIF-1 α.

In an alternate embodiment, the implant of the present invention may be further incorporated with pharmaceutical compounds that reduce inflammation, reduce fibrosis and/or to enhance angiogenesis.

The current invention takes into consideration the unique environment that needs to be established to preserve the ectopic functional viability of transplanted cells or tissue that are optionally incorporated in the implant by the establishment of a highly vascularized environment in a site that is easily accessible, preferably by minimally invasive techniques. Further, it allows the vasculature to come into close proximity to the transplanted cells or tissue providing optimal access to the oxygen and nutrients required to maintain functional viability for prolonged periods of time.

The support of the implant of the present invention is constructed out of a fibrous mat encapsulated by and disposed within a foam. The support contains a plurality of interconnecting spaces within the walls of the support. These spaces form a volume into which therapeutic cells may be placed and allow the in-growth of vasculature into the support. The support is preferably biodegradable. Biodegradable polymers readily break down over time *in vivo* and the biodegraded segments do not elicit a chronic foreign body reaction in the recipient mammal.

The implant of the present invention can be any shape, as long as it is of sufficient size to promote vascularization and incorporate the desired number of therapeutic cells that are optionally incorporated in the implant. The supports of the implants of the present invention can be manufactured by any method known to those of skill in the art, such as, for example, molding, extrusion, weaving and the like.

The support is seeded with cells or tissue prior to transplantation.

Vascularization of a site within a mammal is promoted using the implants disclosed herein. At least one pharmaceutical agent that is either capable of increasing cellular HIF-1α protein or decreasing cellular HIF-1α degradation is incorporated into the support used to construct the implant. Incorporation is achieved by adding an effective amount of the at least one pharmaceutical agent to the raw materials of the support and subsequently fabricating the support. In an alternate embodiment, the support is fabricated and then coated with an effective amount of at least one pharmaceutical agent. The invention also provides an implant of the present invention or an implant produced using the method of the present invention for use in promoting vascularization in a patient by implanting the support into a site within a patient.

### DETAILED DESCRIPTION OF THE INVENTION

The present invention is directed to the biocompatible implant according to claim 1, comprising a support and at least one pharmaceutical agent. The at least one pharmaceutical agent in the implant is capable of increasing cellular HIF-1α protein levels, or of preventing degradation of cellular HIF-1α protein levels, at, or around the implant site. The implants of the present invention are incorporated with therapeutic cells, and the pharmaceutical agent promotes survival of mammalian cells through its ability to stimulate vascularization at, or around the implant.

A biocompatible implant is provided, which is composed of a porous support, loaded with an effective amount of at least one pharmaceutical agent that promotes vascularization of the implant and promotes survival of cells, organoids or organs that are incorporated within the support.

The term "support" as used herein refers to a three-dimensional architecture that is capable of supporting cells on the surface or within the architecture.

The term "porous" as used herein refers to a plurality of inter connecting spaces within the support that enables the distribution of nutrients and cells within the support.

The term "biocompatible" refers to the ability of the support to reside within a mammal so as not to induce toxic or undesirable effects in that mammal.

By "biodegradable" or "absorbable" is meant that the device will be gradually degraded or absorbed by natural biological processes after the device is delivered to a site of interest within a mammal.

The term "implantable" as used herein refers to an implant that is suitable in design, substance and use thereby permitting it to be positioned safely in a mammal. The implant of the present invention can be implanted in a variety of locations in a mammal, particularly where improved, or increased, or new vascularization is desired.

The term "matrix" as used herein refers to the material that comprises the solid component of a support.

The term "therapeutic cell" refers to any cell, organoid or tissue that is employed as an effective treatment of disease or injury.

The term "pharmaceutical agent that increases cellular HIF-1α protein levels" includes chemical agents, growth factors, extracellular matrix proteins, and biologically relevant peptide fragments, that increase cellular HIF-1α protein levels in cells within the implant or around the implant thereby promoting vascularization at or in areas adjacent to the implant site in a mammal.

In a preferred embodiment, the at least one pharmaceutical agent that increases cellular HIF-1α protein levels includes PR-39 (a 39- amino acid macrophage-derived peptide), as, for example, disclosed in WO0130368, hydrazones, as, for example, disclosed in US6660737, anti-fibrotic agents, such as disclosed in WO2003049686 that inhibit 2-oxoglutarate dioxygenase, prolyl hydroxylase inhibitors, such as disclosed in WO2003080566, combinations of the above factors or other agents capable of prolonging the half-life of HIF-1α in cells within and immediately surrounding the implant site.

In an especially preferred embodiment, the prolyl hydroxylase inhibitor is ethyl-3, 4-dihydroxybenzoate, or alternatively, 2,4-diethylpyridine dicarboxylate.

### THE SUPPORT

One of ordinary skill in the art will appreciate that the selection of a suitable material for forming the supports of the present invention depends on several factors. The more relevant factors in the selection of the appropriate material include bioabsorption (or biodegradation) kinetics; *in vivo* mechanical performance; cell response to the material in terms of cell attachment, proliferation, migration and differentiation; and biocompatibility. Other relevant factors, which to some extent dictate the *in vitro* and *in vivo* behavior of the material, include the chemical composition, spatial distribution of the constituents, the molecular weight, the degree of crystallinity, and monomer content in the case of polymeric materials. The surface properties of the materials can also be optimized to achieve the desired hydrophilicity. Exemplary synthetic and natural materials that can be employed in the construction of the supports of the present invention are disclosed in US 5,770,417, US 6,022,743, US 5,567,612, US 5,759,830, US 6,626,950, US 6,534,084, US 6,306,424, US 6,365,149, US 6,599,323, US 6,656,488, and US 6,333,029). Exemplary methods to construct the polymers used in the device of the present invention are disclosed in US patent application US20040062753 A1 and U.S. Pat. No. 4,557,264.

The support is porous and contains at least one pharmaceutical agent. The support is composed of biocompatible fibers (the fibrous component), encapsulated by and disposed within a porous, biocompatible, polymeric matrix (the foam component). The support also includes at least one pharmaceutical agent. The porosity of the support may vary depending on the application and the site of implantation. Porosity can be controlled by a variety of means such as the density of the fibers in the nonwoven component, or the concentration or amount of the polymer solution used in forming the foam component. Cells can attach to both the fibers and to the walls of the matrix encapsulating the fibers. The walls of the matrix incorporate one or more pharmaceutical agents that are locally released at the site of implantation in an effective dose to promote the vascularization of the implanted device and to promote the survival of the incorporated cells.

The supports of the present invention can be biodegradable or non-biodegradable. In the case of a non-biodegradable support, either or both of the fibers and the matrix encapsulating the fibers can be made from non-biodegradable materials, e.g., non-biodegradable polymers including, but not limited to, polyethylene, polyvinyl alcohol (PVA), polymethylmethacrylte (PMMA), silicone, polyethylene oxide (PEO), polyethylene glycol (PEG), and polyurethanes.

Preferably however, the supports of the present invention are biodegradable. Examples of suitable biodegradable materials include biodegradable synthetic polymers such as aliphatic polyesters, polyalkylene oxalates, polyamides, polycarbonates, polyorthoesters, polyoxaesters, polyamidoesters, polyanhydrides and polyphosphazenes.

Aliphatic polyesters are among the preferred biodegradable polymers for use in making the supports according to the present invention. Aliphatic polyesters can be homopolymers or copolymers (random, block, segmented, tapered blocks, graft, triblock, etc.) having a linear, branched or star structure. Suitable monomers for making aliphatic homopolymers and copolymers include, but are not limited to, lactic acid, lactide (including L-, D-, meso and L, D mixtures), glycolic acid, glycolide, ε-caprolactone, p-dioxanone, trimethylene carbonate, polyoxaesters, δ-valerolactone, β-butyrolactone, ε-decalactone, 2,5-diketomorpholine, pivalolactone, α,α-diethylpropiolactone, ethylene carbonate, ethylene oxalate, 3-methyl-1, 4-dioxane-2, 5-dione, 3,3-diethyl-1, 4-dioxan-2, 5-dione, γ-butyrolactone, 1,4-dioxepan-2-one, 1,5-dioxepan-2-one, 6,6-dimethyl-dioxepan-2-one and 6,8-dioxabicycloctane-7-one. These polymers can be readily synthesized by those trained in the art or commercially purchased from various suppliers, such as Birmingham Polymers, Inc (Birmingham, Al).

Elastomeric copolymers are also particularly useful in making the supports of the present invention. Elastomeric supports are less abrasive to the site of implantation as compared to stiff non-elastomeric supports and can be more readily handled at the surgical site. Such polymers are taught in US patent No 6,365,1449. This is particularly advantageous when placing a support in a site such as subcutaneous pouch, omentum, or wrapping the support around an organ such as the pancreas. Suitable elastomeric polymers include those with an inherent viscosity in the range of 1.2 dL/g to 4 dL/g, more preferably 1.2 dL/g to 2 dL/g and most preferably 1.4 dL/g to 2 dL/g, as determined at 25°C in a 0.1 gram per deciliter (g/dL) solution of polymer in hexafluoroisopropanol (HFIP). Further, suitable elastomers exhibit a high percent of elongation and a low modulus, while possessing good tensile strength and good recovery characteristics.

In a preferred embodiment where elastomeric copolymers are used, the elastomer from which the support is formed preferably exhibits a percent elongation greater than 200 percent, and more preferably greater than 500 percent. In addition to these elongation and modulus properties, suitable elastomers also should have a tensile strength greater than 3.5 MPa (500 psi.), preferably greater than 6.9 MPa (1,000 psi.); and a tear strength of greater than 8.9 kg/cm (50 lbs/inch), preferably greater than 14.3 kg/cm (80 lbs/inch).

Exemplary biodegradable, biocompatible elastomers include, but are not limited to, elastomeric copolymers of ε-caprolactone and glycolide with a mole ratio of ε-caprolactone to glycolide of from 35/65 to 65/35, more preferably from 35/65 to 45/55; elastomeric copolymers of ε-caprolactone and lactide where the mole ratio of ε-caprolactone to lactide is from 35/65 to 65/35 and more preferably from 35/65 to 45/55; elastomeric copolymers of lactide and glycolide where the mole ratio of lactide to glycolide is from 95/5 to 85/15; elastomeric copolymers of p-dioxanone and lactide where the mole ratio of p-dioxanone to lactide is from 40/60 to 60/40; elastomeric copolymers of ε-caprolactone and p-dioxanone where the mole ratio of ε-caprolactone to p-dioxanone is from 30/70 to 70/30; elastomeric copolymers of p-dioxanone and trimethylene carbonate where the mole ratio of p-dioxanone to trimethylene carbonate is from 30/70 to 70/30; elastomeric copolymers of trimethylene carbonate and glycolide where the mole ratio of trimethylene carbonate to glycolide is from 30/70 to 70/30; elastomeric copolymers of trimethylene carbonate and lactide where the mole ratio of trimethylene carbonate to lactide is from 30/70 to 70/30, or blends thereof. Exemplary elastomeric polymers and methods to form supports from elastomeric polymers are disclosed in US 6,534,084; US 6,365,149; US6 423,252 and US 6,355,699.

In another embodiment, it is desirable to use polymer blends to form structures which transition from one composition to another composition in a gradient-like architecture. Composite supports having this gradient-like architecture are particularly advantageous in tissue engineering applications to repair or regenerate the structure of naturally occurring tissue. For example, by blending an elastomeric copolymer of ε-caprolactone and glycolide with an elastic copolymer of ε-caprolactone and lactide (e.g., glycolide with an elastic copolymer of ε-caprolactone and lactide (e.g., with a mole ratio of 5/95) a support may be formed that transitions from a softer spongy material to a stiffer more rigid material. Clearly, one of ordinary skill in the art having the benefit of this disclosure will appreciate that other polymer blends may be used for similar gradient effects, or to provide different gradients, e.g. different degradation profiles, stress response profiles or different degrees of elasticity. Such structures have been disclosed in WO02051463.

With a composite support, the fibers encapsulated by a porous matrix are organized as nonwoven mats. Preferably, the fibers are in the form of a nonwoven fibrous mat. Known wet-lay or dry-lay fabrication techniques can be used to prepare the fibrous nonwoven mat of the composite support of the present invention ("Non-woven textiles", by Radko Krcma, Textile Trade Press, Manchester, UK, 1967).

Preferably, the fibers that form the nonwoven fibrous mat of a composite support are made of biodegradable polymers such as polylactic acid (PLA), polyglycolic acid (PGA), ε-polycaprolactone (PCL), polydioxanone (PDO), or copolymers and blends thereof.

The porous matrix of a support as described herein is preferably in the form of a polymeric foam. A foam support or the foam matrix of a composite support can be formed by a variety of techniques well known to those having ordinary skill in the art. For example, the polymeric starting materials may be formed by lyophilization, supercritical solvent foaming, gas injection extrusion, gas injection molding, or casting with an extractable material (e.g., salts, sugar or similar suitable materials).

In one embodiment, the composite support of the present invention is made using a polymer-solvent phase separation technique, such as lyophilization. The steps involved in the preparation of these foams include choosing the appropriate solvents for polymer lyophilization and preparing a homogeneous solution of the polymer in the solvent. The polymer solution is then subjected to a freezing and vacuum-drying cycle. The freezing step phase-separates the polymer solution and the vacuum-drying step removes the solvent by sublimation and/or drying, thus leaving a porous, polymer matrix, or an interconnected, open-cell, porous foam.

Suitable solvents dissolve the biodegradable polymer for forming the foam matrix, and maintain the fibers (e.g., of a nonwoven mat) of the composite support. Exemplary solvents to be matched with the appropriate polymer include, but are not limited to, hexafluoroisopropanol (HFIP), cyclic ethers (e.g., tetrahydrofuran (THF) and dimethylene fluoride (DMF)), acetone, methylethyl ketone (MEK), 1,4-dioxane, dimethlycarbonate, benzene, toluene, N-methyl pyrrolidone, dimethylformamide, chloroform, and mixtures thereof. One of ordinary skill in the art could make the appropriate pairings. Among these solvents, a preferred solvent is 1,4-dioxane. A homogeneous solution of the polymer in the solvent is prepared using standard techniques.

The applicable polymer concentration or amount of solvent to be used may vary, depending on the polymer and the solvent used. In general, the amount of polymer in the solution is in the range of 0.01% to about 90% by weight and, preferably, is in the range of 0.1% to 30% by weight, depending on factors such as the solubility of the polymer in a given solvent and the final properties desired in the foam support including the release profile of the added pharmaceutical agent.

In yet another embodiment of the invention, the foam component or the fibrous component of a support can be chemically cross-linked or combined with hydrogels, such as polyethylene glycol and poly (N-isopropylacryalmide).

In yet another embodiment, the support of the current invention can be combined with a biopolymer selected from the group consisting of hyaluronic acid, collagen, recombinant collagen, cellulose, elastin, alginates, chondroitin sulfate, chitosan, and small intestine submucosa (SIS).

The supports of the present invention preferably include interconnecting pores or voids, which facilitate the incorporation of cells into the support, as well as the transport of nutrients and/or expansion of cells within the support. The interconnected pores preferably range in size from 50 to 1000 µm (microns), preferably 50 to 400 µm (microns), and preferably constitute 70 to 95 percent of the total volume of the support. The range of the pore size in the support can be manipulated by modifying process steps during the preparation of the support.

Support materials have been extensively studied as tissue templates, conduits, barriers, and reservoirs useful for tissue repair. In particular, synthetic and natural materials in the form of foams, sponges, gels, hydrogels, textiles, and nonwoven structures have been used *in vitro* and *in vivo* to reconstruct or regenerate biological tissue, as well as to deliver chemotactic agents for inducing tissue growth, such as the methods disclosed in US 5,770,417, US 6,022,743, US 5,567,612, US 5,759,830, US 6,626,950, US 6,534,084, US 6,306,424, US 6,365,149, US 6,599,323, US 6,656,488, and US 6,333,029). Exemplary polymers used in the device of the present invention are disclosed in US patent application US20040062753 A1 and U.S. Pat. No. 4,557,264.

To form a support incorporated with a pharmaceutical agent, the pharmaceutical agent can be mixed with the polymer solution prior to forming the support. Alternatively, a pharmaceutical agent could be coated onto a fabricated support, preferably in the presence of a pharmaceutical carrier. The pharmaceutical agent may be present as a liquid, a finely divided solid, or any other appropriate physical form. Alternatively, excipients may be added to the support to alter the release rate of the pharmaceutical agent. In an alternate embodiment, the support incorporated with at least one pharmaceutical agent that increases cellular HIF-1 α levels is further incorporated with at least one pharmaceutical compound that is an anti-inflammatory compound, such as, for example compounds disclosed in US 6,509,369.

In a further embodiment, the support incorporated with at least one pharmaceutical agent that increases cellular HIF-1 α levels is further incorporated with at least one pharmaceutical compound that is an anti-apoptotic compound, such as, for example compounds disclosed in US 6,793,945.

In a further embodiment, the support incorporated with at least one pharmaceutical agent that increases cellular HIF-1 α levels is further incorporated with at least one pharmaceutical compound that is an inhibitor of fibrosis, such as, for example compounds disclosed in US 6,331,298.

In a further embodiment, the support incorporated with at least one pharmaceutical agent that increases cellular HIF-1 α levels is further incorporated with at least one pharmaceutical compound that is capable of enhancing angiogenesis, such as, for example compounds disclosed in US20040220393 and US20040209901.

In a further embodiment, the support incorporated with at least one pharmaceutical agent that increases cellular HIF-1 α levels is further incorporated with at least one pharmaceutical compound that is an immunosuppressive compound, such as, for example compounds disclosed in US20040171623.

In a further embodiment, the support incorporated with at least one pharmaceutical agent that increases cellular HIF-1 α levels is further incorporated with at least one pharmaceutical compound that is a growth factor, such as, for example members of the TGF-β family, including TGF-β1, 2, and 3, bone morphogenic proteins (BMP-2, -3, -4, -5, -6, -7, -11, -12, and -13), fibroblast growth factors-1 and -2, platelet-derived growth factor-AA, and -BB, platelet rich plasma, insulin growth factor (IGF-I, II) growth differentiation factor (GDF-5, -6, -8, -10, -15) vascular endothelial cell-derived growth factor (VEGF), pleiotrophin, endothelin. Other pharmaceutical compounds can include, for example, nicotinamide, hypoxia inducible factor 1-alpha, glucagon like peptide-I (GLP-1) and II, Exendin-4, retinoic acid, parathyroid hormone, tenascin-C, tropoelastin, thrombin-derived peptides, cathelicidins, defensins, laminin, biological peptides containing cell- and heparin-binding domains of adhesive extracellular matrix proteins such as fibronectin and vitronectin, MAPK inhibitors, such as, for example, compounds disclosed in US20040209901 and US20040132729.

In an alternate embodiment, the support incorporated with at least one pharmaceutical agent that decreases the degradation of HIF-1 α protein is further incorporated with at least one pharmaceutical compound that is an anti-inflammatory compound, such as, for example compounds disclosed in US 6,509,369.

In a further embodiment, the support incorporated with at least one pharmaceutical agent that decreases the degradation of HIF-1 α protein is further incorporated with at least one pharmaceutical compound that is an anti-apoptotic compound, such as, for example compounds disclosed in US 6,793,945.

In a further embodiment, the support incorporated with at least one pharmaceutical agent that decreases the degradation of HIF-1 α protein is further incorporated with at least one pharmaceutical compound that is an inhibitor of fibrosis, such as, for example compounds disclosed in US 6,331,298.

In a further embodiment, the support incorporated with at least one pharmaceutical agent that decreases the degradation of HIF-1 α protein is further incorporated with at least one pharmaceutical compound that is capable of enhancing angiogenesis, such as, for example compounds disclosed in US20040220393 and US20040209901.

In a further embodiment, the support incorporated with at least one pharmaceutical agent that decreases the degradation of HIF-1 α protein is further incorporated with at least one pharmaceutical compound that is an immunosuppressive compound, such as, for example compounds disclosed in US20040171623.

In a further embodiment, the support incorporated with at least one pharmaceutical agent that decreases the degradation of HIF-1 α protein is further incorporated with at least one pharmaceutical compound that is a growth factor, such as, for example members of the TGF-β family, including TGF-β1, 2, and 3, bone morphogenic proteins (BMP-2, -3, -4, -5, -6, -7, -11, -12, and -13), fibroblast growth factors-1 and -2, platelet-derived growth factor-AA, and -BB, platelet rich plasma, insulin growth factor (IGF-I, II) growth differentiation factor (GDF-5, -6, -8, -10, -15) vascular endothelial cell-derived growth factor (VEGF), pleiotrophin, endothelin. Other pharmaceutical compounds can include, for example, nicotinamide, hypoxia inducible factor 1-alpha, glucagon like peptide-I (GLP-1) and II, Exendin-4, retinoic acid, parathyroid hormone, tenascin-C, tropoelastin, thrombin-derived peptides, cathelicidins, defensins, laminin, biological peptides containing cell- and heparin-binding domains of adhesive extracellular matrix proteins such as fibronectin and vitronectin, MAPK inhibitors, such as, for example, compounds disclosed in US20040209901 and US20040132729.

In a preferred embodiment, the pharmaceutical agent that is capable of increasing cellular HIF-1α levels is a prolyl hydoxylase inhibitor, incorporated into the walls of a support of the present invention. In a most preferred embodiment, the prolyl hydoxylase inhibitor is ethyl-3,4-dihydroxybenzoate (Aldrich, USA; catalogue# E2485-9) or 2,4-diethylpyridinedicarboxylate (Cayman chemical company, USA; catalogue# 71200).

In a preferred embodiment, the pharmaceutical agent that is capable of decreasing the degradation of HIF-1α protein is a prolyl hydoxylase inhibitor, incorporated into the walls of a support of the present invention. In a most preferred embodiment, the prolyl hydoxylase inhibitor is ethyl-3,4-dihydroxybenzoate (Aldrich, USA; catalogue# E2485-9) or 2,4-diethylpyridinedicarboxylate (Cayman chemical company, USA; catalogue# 71200).

The amount of the prolyl hydoxylase inhibitor effective to promote angiogenesis or up regulation of genes involved in angiogenesis may vary, depending on the support used and the nature of the inhibitor, and can be readily determined by one skilled in the art. Generally speaking, an effective amount of a prolyl hydoxylase inhibitor is 1 pico gram/cm² to 1 mg/cm² of the polymeric matrix, and more typically 1 pico gram/cm² to 10 µg/cm² of the polymeric matrix.

The biodegradable supports of the present invention can undergo gradual degradation (mainly through hydrolysis) with concomitant release of the dispersed pharmaceutical agent for a sustained or extended period that is sufficient to promote vascularization of the implant or the area around the implant. Preferably the implant promotes prolonged delivery of the at least one pharmaceutical agent, e.g. over 1 to 5,000 hours, preferably 2 to 800 hours, of effective amounts, e.g. 0.0001 mg/kg/hour to 10 mg/kg/hour, of the pharmaceutical agent. This dosage form can be administered as is necessary depending on the subject being treated, the severity of the affliction, the judgment of the prescribing physician, and the like. Following this or similar procedures, those skilled in the art will be able to prepare a variety of formulations.

### THERAPEUTIC USE OF THE IMPLANTS

The present invention provides an implant, comprising a support that has incorporated into it at least one pharmaceutical agent that is capable of promoting vascularization. The implant of the present invention is thought to promote vascularization by increasing HIF-1α protein levels at, or adjacent to the implant site, or by decreasing the degradation of HIF-1α at, or adjacent to the implant site.

The support of the present invention can be implanted in an animal. The implant contains therapeutic cells or tissues that are incorporated into the support prior to implantation. The cells may be cultured under standard conditions known to those skilled in the art in order to increase the number of cells or induce differentiation to the desired phenotype prior to seeding into the support. Alternatively, the cells can be injected directly into the support and then cultured *in vitro* under conditions that promote proliferation and deposition of the appropriate biological matrix prior to implantation. One skilled in the art can readily recognize such conditions.

To introduce the cells into a support, the support is contacted and incubated with a suspension containing the cells, or clusters of cells. The incubation can be performed for a short period of time (< 1 day) just prior to implantation, or for longer a period (> 1 day) to allow for enhanced cell attachment, cell proliferation and extracellular matrix synthesis within the seeded support prior to implantation.

The effects of the implants of the present invention on cellular levels of HIF-1α protein and mRNA levels can be assayed in a variety of ways known in the art. For example, HIF-1α mRNA levels can be quantitated by, e.g., Northern blot analysis, competitive polymerase chain reaction (PCR), or real-time PCR (RT-PCR). Real-time quantitative PCR is presently preferred. RNA analysis can be performed on total cellular RNA or poly(A)+ mRNA. Methods of RNA isolation are well known in the art. Northern blot analysis is also routine in the art. Real- time quantitative (PCR) can be conveniently accomplished using the commercially available ABI PRISM® 7600, 7700, or 7900 Sequence Detection System, available from PE-Applied Biosystems, Foster City, Calif. and used according to manufacturer's instructions.

Protein levels of HIF-1α can be quantitated in a variety of ways well known in the art, such as immunoprecipitation, Western blot analysis (immunoblotting), enzyme-linked immunosorbent assay (ELISA) or fluorescence-activated cell sorting (FACS). Antibodies directed to HIF-1α can be identified and obtained from a variety of sources, such as the MSRS catalog of antibodies (Aerie Corporation, Birmingham, Mich.), or can be prepared via conventional monoclonal or polyclonal antibody generation methods well known in the art. Methods for the analysis of the levels of HIF-1α protein and mRNA are disclosed in US 6,020,462.
The invention also provides an implant of the present invention or an implant produced using the method of the present invention for use in promoting vascularization in a patient by implanting the support into a site within a patient.

The site where the device can be implanted can be any clinically relevant site, such as, for example, the liver, the natural pancreas, the renal subcapsular space, the mesentery, the omentum, a subcutaneous pocket, or the peritoneum. The site can be an immunologically privileged site, either naturally existing or created using, for example, Sertoli cells.

The therapeutic cells useful for administration include autologous, allogeneic, or xenogeneic cells. In the case that the implant is for use to treat diabetes, the cells can be stem cells, pancreatic precursor/progenitor cells, genetically engineered insulin producing cells, primary or expanded partially or fully differentiated islets or insulin producing cells.

Such treatment may also be used for other types of cell therapy, including, for example, hepatocytes for the treatment of liver failure, chromaffin cells for chronic pain, cells that produce clotting factors for hemophilia, and cells that produce nerve growth factors for neurodegenerative disease such as Parkinson's or Alzheimer's disease, as well as fibroblasts, myofibroblasts, cardiovascular cells, neural cells, and neural precursor cells.

Other cells that can be therapeutically effective for different applications include, but are not limited to, progenitor cells, precursor cells, stem cells, bone marrow cells, umbilical cord blood cells, angioblasts, endothelial cells, osteoblasts, smooth muscle cells, kidney cells, fibroblasts, myofibroblasts, cardiovascular cells, neural cells, neural precursor cells, amniotic cells and post-partum placental cells. The cells may be genetically engineered to produce a therapeutic protein, or to down-regulate the recipient's immune response.

The cells introduced into the implant of the present invention need not be limited to just one cell type. The implants of the present invention enable the construction of "artificial organs", wherein the cell types found in normal organs, such as, for example, the liver, the pancreas, the kidney, are incorporated into the support of the implant. In an alternative embodiment, cells that are capable of modulating the recipient's immune response, such as, for example, mesenchymal stem cells and suppressor T cells, as disclosed in US 6,328,960, US 6,281,012 and US 6,685,936.

### REFERENCE EXAMPLES

The following examples are illustrative of the principles and practice of the invention and are not intended to limit the scope of the invention. In the examples, the polymers and monomers were characterized in chemical composition and purity (NMR, FTIR), thermal analysis (DSC) and molecular weight by conventional analytical techniques.

Inherent viscosities (I.V., dL/g) of the polymers and copolymers were measured using a 50 bore Cannon-Ubbelhode dilution viscometer (Labovisco, Zoetermeer, Netherlands) immersed in a thermostatically controlled water bath at 30°C utilizing chloroform or hexafluoroisopropanol (HFIP) as the solvent at a concentration of 0.1 g/dL.

In these examples certain abbreviations are used. These include PCL to indicate polymerized ε-caprolactone and PGA to indicate polymerized glycolide. Additionally, the ratios in front of the copolymer identification indicate the respective mole percentages of each constituent.

### REFERENCE EXAMPLE 1: FABRICATION OF A SUPPORT CONTAINING AT LEAST ONE PHARMACEUTICAL AGENT.

The supports used to form the foam component of the implants of the present invention can be any size or shape. The shape is dictated by the mold used to form the support, which was custom-made for this example by Hi-Tech Machine and Design LLC, Flemington, NJ, and is not intended to limit the scope of the invention.

The polymer used to manufacture the foam component was a 35/65 PCL/PGA copolymer produced by Birmingham Polymers Inc. (Birmingham, AL), with an I.V. of 1.45 dL/g. A 5/95 weight ratio of 35/65 PCL/PGA in 1,4-dioxane solvent was weighed out. The polymer and solvent were placed into a flask, which in turn was put into a water bath and stirred for 5 hours at 70°C to form a solution. The solution then was filtered using an extraction thimble (extra coarse porosity, type ASTM 170-220 (EC)) and stored in a flask. Next HIF-1α hydroxylase inhibitor (ethyl-3, 4-dihydroxybenzoate, (Aldrich)) was added at the concentrations listed in Table 1.

A laboratory scale lyophilizer, or freeze dryer, (Model Duradry, FTS Kinetics, Stone Ridge, NY), was used to form the foam support. The polymer solution was added into a 10.2 cm by 10.2 cm (4-inch by 4-inch) aluminum mold to a height of 2 mm. The mold assembly then was placed on the shelf of the lyophilizer and the freeze dry sequence begun. The freeze drying sequence used in this example was: 1) -17°C for 60 minutes, 2) -5°C for 60 minutes under vacuum 100 mT, 3) 5°C for 60 minutes under vacuum 20 mT, 4) 20°C for 60 minutes under vacuum 20 mT.

After the cycle was completed, the mold assembly was taken out of the freeze dryer and allowed to degas in a vacuum hood for 2 to 3 hours. The foam support was stored under nitrogen. The pore size of this composite support was determined using Mercury Porosimetry analysis (J.C. Groen and L.A.A. Peffer, "Influence of dead space measurement on adsorption characteristics of microporous zeolites", The MicroReport, 3rd quarter 1997, Vol. 8, No. 3, p.8.). The range of pore size was 1-300 µm with a median pore size of 45 µm.

The resulting supports contained the HIF-1α hydroxylase inhibitor entrapped in the walls of the polymeric foam. The thickness of the support was approximately 1.5 mm.

### REFERENCE EXAMPLE 2: FABRICATION OF A COMPOSITE SUPPORT CONTAINING AT LEAST ONE PHARMACEUTICAL AGENT.

In this example, a support was prepared from two parts: 1) a foam component and 2) a non-woven mat component.

The nonwoven mat was made as follows: A copolymer of PGA/PLA (90/10) (Ethicon, USA) was melt-extruded into continuous multifilament yarn by conventional methods of making yarn and subsequently oriented in order to increase strength, elongation and energy required to rupture. The yarns comprised filaments of approximately 20 µm (microns) in diameter. These yarns were then cut and crimped into uniform 5.1 cm (2-inch) lengths to form 5.1 cm (2-inch) staple fiber.

A dry lay needle-punched nonwoven mat was then prepared utilizing the 90/10 PGA/PLA copolymer staple fibers. The staple fibers were opened and carded on standard nonwoven machinery. The resulting mat was in the form of webbed staple fibers. The webbed staple fibers were needle punched to form the dry lay needle-punched, fibrous nonwoven mat. The mat was scoured with isopropanol for 60 minutes, followed by drying under vacuum. The resulting mat was approximately 2mm thick.

The polymer used to manufacture the foam component was a 35/65 PCL/PGA copolymer produced by Birmingham Polymers Inc. (Birmingham, AL), with an I.V. of 1.45 dL/g. A 0.5/99.5 weight ratio of 35/65 PCL/PGA in 1,4-dioxane solvent was weighed out. The polymer and solvent were placed into a flask, which in turn was put into a water bath and stirred for 5 hours at 70 °C to form a solution. The solution then was filtered using an extraction thimble (extra coarse porosity, type ASTM 170-220 (EC)) and stored in a flask. Next, the HIF-1α hydroxylase inhibitor (ethyl-3, 4-dihydroxybenzoate, (Aldrich)) was added at the concentrations shown in Table 1.

A laboratory scale lyophilizer, or freeze dryer, (Model Duradry, FTS Kinetics, Stone Ridge, NY), was used to form the composite support. Approximately 10 ml of the polymer solution containing the HIF-1α hydroxylase inhibitor was added into a 4-inch by 4-inch aluminum mold to cover uniformly the mold surface. The needle-punched nonwoven mat was immersed into the beaker containing the rest of the solution until fully soaked and then was positioned it in the aluminum mold. The remaining polymer solution was poured into the mold so that the solution covered the nonwoven mat and reached a height of 2 mm in the mold.

The mold assembly then was placed on the shelf of the lyophilizer and the freeze dry sequence begun. The freeze dry sequence used in this example was: 1) -17 °C for 60 minutes, 2) -5 °C for 60 minutes under vacuum (100 mT), 3) 5 °C for 60 minutes under vacuum (20 mT), 4) 20 °C for 60 minutes under vacuum (20 mT).

After the cycle was completed, the mold assembly was taken out of the freeze dryer and allowed to degas in a vacuum hood for 2 to 3 hours. The foam support was stored under nitrogen. The pore size of this composite support was determined using Mercury Porosimetry analysis. The range of pore size was 1-300 µm with a median pore size of 45 µm. The resulting supports contained the HIF-1αhydroxylase inhibitor entrapped in the walls of the polymeric foam. The thickness of the support was approximately 1.5 mm.

### REFERENCE EXAMPLE 3: CHARACTERIZATION OF SUPPORT LOADED WITH A HIF-1 ALPHA HYDROXYLASE INHIBITOR

8 mm discs were prepared from the foam sheet described in Example 1 and placed in a 2 ml glass vial containing 0.5 ml of DI-water. The vial was then sealed and placed on a shaker overnight to ensure complete release of the drug content from the support into the solvent. The following day the support was removed from the vial and the solution was analyzed via HPLC to determine the total drug content. A C-18 column was used with a mobile phase composed of 50% methanol, and 50% water (pH=2.7; H₂SO₄). The flow rate was controlled at 1 ml/min. The injection volume was 10µl and detection was done at 254nm. The run time of the method was 30 minutes. A stock solution was prepared by dissolving the drug in pure water and stored in the refrigerator in an amber flask. The standard solutions were prepared by serial dilution of the stock solution. The calibration curve was found to be linear in the range of 5-100µg/ml. Linearity and reproducibility were assessed by duplicate injections of four standards. Table I shows the content of the agent in the support as a function of initial concentration in polymer solution.

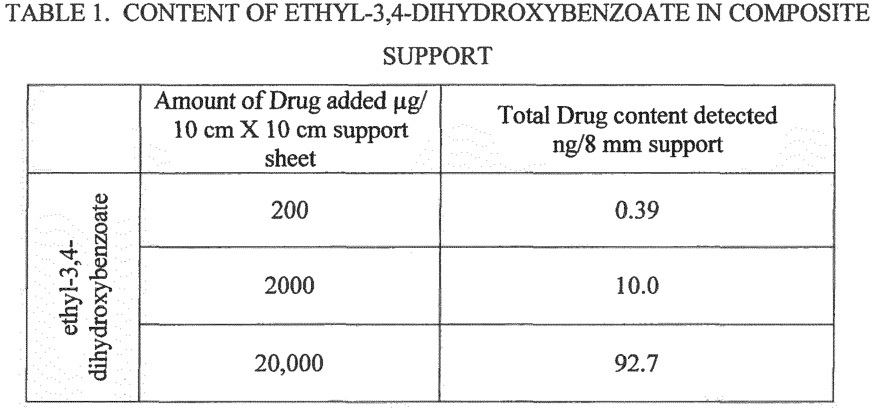

## Claims

1. A biocompatible implant, comprising a support, at least one pharmaceutical agent that either increases cellular HIF-1α protein levels or inhibits the degradation of HIF-1α protein and mammalian tissue attached to, or incorporated within the support; wherein the support is a fibrous mat encapsulated by and disposed within a foam.

2. The implant of claim 1, wherein the support is biodegradable.

3. The implant of claim 1 or claim 2, wherein the at least one pharmaceutical agent is incorporated into the support prior to the formation of the support by adding the at least one pharmaceutical agent into a polymer solution for forming the support.

4. The implant of claim 1 or claim 2, wherein the at least one pharmaceutical agent is incorporated into the support after the formation of the support by coating the support with the at least one pharmaceutical agent.

5. The implant of any one of claims 1-4, wherein the mammalian tissue comprises cells, tissue isolated from an organ or an isolated organ.

6. The implant of any one of claims 1 to 5, wherein the at least one pharmaceutical agent is a HIF-1α hydroxylase inhibitor.

7. The implant of claim 6, wherein the HIF-1α hydroxylase inhibitor is 2,4-diethylpyridinedicarboxylate or ethyl-3,4-dihydroxybenzoate.

8. The implant of any of claims 1-7, wherein the fibrous mat of the support is made by a wet-lay or dry-lay procedure.

9. The implant of any of claims 1-8, wherein the foam of the support is formed by a polymer-solvent phase separation technique, supercritical solvent foaming, gas injection extrusion, gas injection molding or casting with an extractable material.

10. The implant of any one of claims 1 to 9, wherein the support is made of one or more biodegradable polymers.

11. The implant of claim 10, wherein the polymer is hyaluronic acid, collagen, recombinant collagen, cellulose, elastin, an alginate, chondroitin sulfate, chitosan, chitin, keratin, silk, small intestine submucosa (SIS) or a combination thereof.

12. The implant of any one of claims 1 to 9, wherein the support is made from one or more synthetic polymers.

13. The implant of claim 12, wherein the polymer is an aliphatic polyester, polyalkylene oxalate, polyamide, polycarbonate, polyorthoester, polyoxaester, polyamidoester, polyanhydride or polyphosphazene.

14. The implant of claim 13, wherein the polymer is an aliphatic polyester which is a homopolymer or copolymer of lactic acid, lactide, glycolic acid, glycolide, ε-caprolactone, p-dioxanone, trimethylene carbonate, polyoxaesters, δ-valerolactone, β-butyrolactone, ε-decalactone, 2,5-diketomorpholine, pivalolactone, α,α-diethylpropiolactone, ethylene carbonate, ethylene oxalate, 3-methyl-1,4-dioxane-2,5-dione, 3,3-diethyl-1,4-dioxan-2,5-dione, γ-butyrolactone, 1,4-dioxepan-2-one, 1,5-dioxepan-2-one, 6,6-dimethyl-dioxepan-2-one or 6,8-dioxabicycloctane-7-one.

15. The implant of claim 12, wherein the polymer is an elastomer.

16. The implant of claim 15, wherein the elastomer is a copolymer of: ε-caprolactone and glycolide, ε-caprolactone and lactide, lactide and glycolide, p-dioxanone and lactide, ε-caprolactone and p-dioxanone, p-dioxanone and trimethylene carbonate, trimethylene carbonate and glycolide, trimethylene carbonate and lactide or a combination thereof.

17. The implant of claim 12, wherein the material comprising the support has a gradient structure, **characterized by** a continuous transition from a first biodegradable polymer composition to a second biodegradable polymer composition.

18. A method of making a biocompatible implant to increase cellular HIF-1α levels or to decrease the degradation of HIF-1α protein, comprising the steps of:
a. incorporating at least one pharmaceutical agent capable of increasing cellular HIF-1α protein levels or decreasing the degradation of HIF-1α protein into a support, wherein the support is a fibrous mat encapsulated by and disposed within a foam; and
b. introducing the mammalian tissue into the support.

19. The method of claim 18, wherein the support is further treated with at least one of a growth factor, an anti-rejection agent, an analgesic, an anti-oxidant, an anti-apoptotic agent, an anti-inflammatory agent or an immunosuppressive drug.

20. An implant of any one of claims 1 to 17 or an implant produced using the method of claim 18 or claim 19 for use in promoting vascularization in a patient by implanting the support into a site within a patient.

21. The implant for use of claim 20, wherein the site is the liver, the natural pancreas, the renal subcapsular space, the mesentery, the omentum, a subcutaneous pocket, or the peritoneum.

## Patentansprüche

1. Biokompatibles Implantat, umfassend einen Träger, wenigstens ein pharmazeutisches Mittel, das entweder die zellulären HIF-1α-Proteinspiegel erhöht oder den Abbau von HIF-1α-Protein und Säugergewebe, das an dem Träger haftet oder darin einverleibt ist, hemmt; wobei der Träger ein Faservlies ist, das von einem Schaumstoff verkapselt und darin angeordnet ist.

2. Implantat gemäß Anspruch 1, wobei der Träger biologisch abbaubar ist.

3. Implantat gemäß Anspruch 1 oder Anspruch 2, wobei das wenigstens eine pharmazeutische Mittel vor dem Bilden des Trägers durch Zugeben des wenigstens einen pharmazeutischen Mittels in eine Polymerlösung zum Herstellen des Trägers in den Träger einverleibt worden ist.

4. Implantat gemäß Anspruch 1 oder Anspruch 2, wobei das wenigstens eine pharmazeutische Mittel nach dem Bilden des Trägers durch Beschichten des Trägers mit dem wenigstens einen pharmazeutischen Mittel in den Träger einverleibt worden ist.

5. Implantat gemäß einem der Ansprüche 1-4, wobei das Säugergewebe Zellen, aus einem Organ isoliertes Gewebe oder ein isoliertes Organ umfasst.

6. Implantat gemäß einem der Ansprüche 1 bis 5, wobei das wenigstens eine pharmazeutische Mittel ein HIF-1α-Hydroxylasehemmer ist.

7. Implantat gemäß Anspruch 6, wobei der HIF-1α-Hydroxylasehemmer 2,4-Diethylpyridindicarboxylat oder Ethyl-3,4-dihydroxybenzoat ist.

8. Implantat gemäß einem der Ansprüche 1-7, wobei das Faservlies des Trägers durch ein Naßlege- oder Trockenlegeverfahren hergestellt ist.

9. Implantat gemäß einem der Ansprüche 1-8, wobei der Schaumstoff des Trägers durch ein Polymer-Lösungsmittel-Phasentrennungsverfahren, Schäumen mit überkritischem Lösungsmittel, Extrusion mit Gasinjektion, Formen mit Gasinjektion oder Gießen mit einem extrahierbaren Material hergestellt ist.

10. Implantat gemäß einem der Ansprüche 1 bis 9, wobei der Träger aus einem oder mehreren biologisch abbaubaren Polymeren besteht.

11. Implantat gemäß Anspruch 10, wobei das Polymer Hyaluronsäure, Collagen, rekombinantes Collagen, Cellulose, Elastin, ein Alginat, Chondroitinsulfat, Chitosan, Chitin, Keratin, Seide, Dünndarmsubmukosa (SIS) oder eine Kombination davon ist.

12. Implantat gemäß einem der Ansprüche 1 bis 9, wobei der Träger aus einem oder mehreren synthetischen Polymeren besteht.

13. Implantat gemäß Anspruch 12, wobei das Polymer ein aliphatischer Polyester, Polyalkylenoxalat, Polyamid, Polycarbonat, Polyorthoester, Polyoxaester, Poylamidoester, Polyanhydrid oder Polyphosphazen ist.

14. Implantat gemäß Anspruch 13, wobei das Polymer ein aliphatischer Polyester ist, der ein Homopolymer oder Copolymer von Milchsäure, Lactid, Glycolsäure, Glycolid, ε-Caprolacton, p-Dioxanon, Trimethylencarbonat, Polyoxaestern, δ-Valerolacton, β-Butyrolacton, ε-Decalacton, 2,5-Diketomorpholin, Pivalolacton, α,α-Diethylpropiolacton, Ethylencarbonat, Ethylenoxalat, 3-Methyl-1,4-dioxan-2,5-dion, 3,3-Diethyl-1,4-dioxan-2,5-dion, γ-Butyrolacton, 1,4-Dioxepan-2-on, 1,5-Dioxepan-2-on, 6,6-Dimethyldioxepan-2-on oder 6,8-Dioxabicyclooctan-7-on ist.

15. Implantat gemäß Anspruch 12, wobei das Polymer ein Elastomer ist.

16. Implantat gemäß Anspruch 15, wobei das Elastomer ein Copolymer von: ε-Caprolacton und Glycolid, ε-Caprolacton und Lactid, Lactid und Glycolid, p-Dioxanon und Lactid, ε-Caprolacton und p-Dioxanon, p-Dioxanon und Trimethylencarbonat, Trimethylencarbonat und Glycolid, Trimethylencarbonat und Lactid oder eine Kombination davon ist.

17. Implantat gemäß Anspruch 12, wobei das Material, das den Träger umfasst, eine Gradientenstruktur aufweist, die durch einen kontinuierlichen Übergang von einer ersten biologisch abbaubaren Polymerzusammensetzung zu einer zweiten biologisch abbaubaren Polymerzusammensetzung gekennzeichnet ist.

18. Verfahren zum Herstellen eines biokompatiblen Implantats zum Erhöhen des zellulären HIF-1α-Spiegels oder zum Verringern des Abbaus von HIF-1α-Protein, umfassend die Schritte:
a. Einverleiben wenigstens eines pharmazeutischen Mittels, das fähig ist, den zellulären HIF-1α-Proteinspiegel zu erhöhen oder den Abbau von HIF-1α-Protein zu verringern, in einen Träger, wobei der Träger ein Faservlies ist, das von einem Schaumstoff verkapselt und darin angeordnet ist; und
b. Einführen des Säugergewebes in den Träger.

19. Verfahren gemäß Anspruch 18, wobei der Träger ferner mit wenigstens einem von einem Wachstumsfaktor, einem Antiabstoßungsmittel, einem Analgetikum, einem Antioxidationsmittel, einem Antiapoposemittel, einem Entzündungshemmer und einem immunsuppressiven Arzneimittel behandelt wird.

20. Implantat gemäß einem der Ansprüche 1 bis 17 oder Implantat, das unter Verwendung des Verfahrens gemäß Anspruch 18 oder Anspruch 19 hergestellt ist, für die Verwendung zum Fördern der Vaskularisation bei einem Patienten durch Implantieren des Trägers an einen Ort in einem Patienten.

21. Implantat für die Verwendung gemäß Anspruch 20, wobei der Ort die Leber, die natürliche Pankreas, der renale subkapsuläre Raum, das Mesenterium, das Omentum, eine subkutane Tasche oder das Peritoneum ist.

## Revendications

1. Implant biocompatible, comprenant un support, au moins un agent pharmaceutique qui augmente les niveaux cellulaires de protéine HIF-1α ou inhibe la dégradation de la protéine HIF-1α et des tissus mammaliens qui sont liés au support ou y sont incorporés ; où le support est un tapis de fibres encapsulé dans une mousse et disposé à l'intérieur de celle-ci.

2. Implant selon la revendication 1, où le support est biodégradable.

3. Prothèse selon la revendication 1 ou la revendication 2, où l'au moins un agent pharmaceutique est incorporé au support avant la formation du support par ajout de l'au moins un agent pharmaceutique dans une solution polymère pour former le support.

4. Implant selon la revendication 1 ou la revendication 2, où l'au moins un agent pharmaceutique est incorporé au support après la formation du support par revêtement du support avec l'au moins un agent pharmaceutique.

5. Implant selon l'une quelconque des revendications 1 à 4, où les tissus mammaliens sont constitués de cellules, de tissus isolés d'un organe ou d'un organe isolé.

6. Implant selon l'une quelconque des revendications 1 à 5, où l'au moins un agent pharmaceutique est un inhibiteur de HIF-1α hydroxylase.

7. Implant selon la revendication 6, où l'inhibiteur de HIF-1α hydroxylase est le pyridine-2,4-dicarboxylate de diéthyle ou le 3,4-dihydroxybenzoate d'éthyle.

8. Implant selon l'une quelconque des revendications 1 à 7, où le tapis fibreux du support est fabriqué par une procédure de dépôt humide ou de dépôt sec.

9. Implant selon l'une quelconque des revendications 1 à 8, où la mousse du support est formée par une technique de séparation de phase polymère-solvant, expansion en solvant supercritique, extrusion par injection de gaz, moulage par injection de gaz ou coulage avec un matériau extractible.

10. Implant selon l'une quelconque des revendications 1 à 9, où le support est fabriqué d'un ou de plusieurs polymères biodégradables.

11. Implant selon la revendication 10, où le polymère est choisi parmi acide hyaluronique, collagène, collagène recombinant, cellulose, élastine, un alginate, sulfate de chondroïtine, chitosane, chitine, kératine, soie, sous-muqueuse de l'intestin grêle ou l'une de leurs combinaisons.

12. Implant selon l'une quelconque des revendications 1 à 9, où le support est fabriqué à partir d'un ou de plusieurs polymères synthétiques.

13. Implant selon la revendication 12, où le polymère est un polyester aliphatique, un polyalkylène oxalate, un polyamide, un polycarbonate, un polyorthoester, un polyoxaester, un polyamidoester, un polyanhydride ou un polyphosphazène.

14. Implant selon la revendication 13, où le polymère est un polyester aliphatique qui est un homopolymère ou un copolymère d'acide lactique, lactide, acide glycolique, glycolide, ε-caprolactone, p-dioxanone, carbonate de triméthylène, polyoxaesters, δ-valérolactone, β-butyrolactone, ε-décalactone, 2,5-dikétomorpholine, pivalolactone, α,α-diéthylpropiolactone, carbonate d'éthylène, oxalate d'éthylène, 3-méthyl-1,4-dioxane-2,5-dione, 3,3-diéthyl-1,4-dioxan-2,5-dione, γ-butyrolactone, 1,4-dioxépan-2-one, 1,5-dioxépan-2-one, 6,6-diméthyl-dioxépan-2-one ou 6,8-dioxa-bicycloctane-7-one.

15. Implant selon la revendication 12, où le polymère est un élastomère.

16. Implant selon la revendication 15, où l'élastomère est un copolymère de : ε-caprolactone et glycolide, ε-caprolactone et lactide, lactide et glycolide, p-dioxanone et lactide, ε-caprolactone et p-dioxanone, p-dioxanone et carbonate de triméthylène, carbonate de triméthylène et glycolide, carbonate de triméthylène et lactide ou l'une de leurs combinaisons.

17. Implant selon la revendication 12, où le matériau constituant le support présente une structure en gradient, **caractérisée par** une transition continue depuis une première composition de polymère biodégradable vers une seconde composition de polymère biodégradable.

18. Procédé de fabrication d'un implant biocompatible destiné à augmenter les niveaux cellulaires de HIF-1α ou à diminuer la dégradation de la protéine HIF-1α, comprenant les étapes consistant à :
a. incorporer dans un support au moins un agent pharmaceutique capable d'augmenter les niveaux cellulaires de protéines HIF-1α ou de diminuer la dégradation de la protéine HIF-1α, où le support est un tapis fibreux encapsulé par une mousse et disposé à l'intérieur de celle-ci ; et
b. introduire les tissus mammaliens dans le support.

19. Procédé selon la revendication 18, où le support est en outre traité par au moins l'un des membres du groupe constitué par un facteur de croissance, un agent anti-rejet, un analgésique, un antioxydant, un agent anti-apoptotique, un agent anti-inflammatoire ou un médicament immunosuppresseur.

20. Implant selon l'une quelconque des revendications 1 à 17 ou implant produit en utilisant le procédé selon la revendication 18 ou la revendication 19 pour utilisation dans la promotion de la vascularisation chez un patient par implantation du support dans un site situé chez le patient.

21. Implant pour utilisation selon la revendication 20, où le site est le foie, le pancréas naturel, l'espace sous-capsulaire rénal, le mésentère, l'omentum, une poche sous-cutanée ou le péritoine.
